# EUROPEAN PATENT SPECIFICATION

(11) **EP 2 500 106 B1**
(45) Date of publication and mention of the grant of the patent: **20.09.2017**
(21) Application number: 10829914.0
(22) Date of filing: 09.11.2010
(51) Int. Cl.: B05B 17/06, A61M 11/00, C02F 1/46, A61M 15/02

(54) **SURFACE ACOUSTIC WAVE ATOMIZATION DEVICE**
VORRICHTUNG ZUR ATOMISIERUNG AKUSTISCHER OBERFLÄCHENWELLEN
DISPOSITIF DE PULVÉRISATION À ONDES ACOUSTIQUES DE SURFACE

(30) Priority: 11.11.2009 JP 2009258091
(43) Date of publication of application: 19.09.2012
(73) Proprietor: Panasonic Corporation, Kadoma-shi Osaka 571-8501 (JP)
(72) Inventor: ISHIGAMI, Youhei, Kadoma-shi Osaka 571-8686 (JP); OKANO, Masanori, Kadoma-shi Osaka 571-8686 (JP)
(74) Representative: Appelt, Christian W.
(86) International application number: PCT/JP2010/069881
(87) International publication number: WO 2011/058955

(56) References cited:
- WO-A1-2009/145099
- JP-A- 2003 136 005
- JP-A- 2008 036 450
- JP-A- 2008 104 974
- JP-A- 2009 041 882
- JP-A- 2009 168 329
- JP-A- 2010 005 606

## Description

### TECHNICAL FIELD

The present invention relates to an atomization device which uses a surface acoustic wave.

### BACKGROUND ART

Conventionally, a phenomenon is known that if a liquid is supplied to a surface of a substrate consisting of piezoelectric material etc., on which a surface acoustic wave is propagating, the liquid receives energy of the surface acoustic wave, and then flows or vibrates, and flies as micro-particles. Devices which atomize a liquid by using this phenomenon have been proposed variously. Among these atomization devices using the surface acoustic wave, one surface acoustic wave atomization device is known which is equipped with a liquid film formation component for forming a film formation gap to distribute a supplied liquid in a shape of a film on a surface of a substrate (for example, refer to patent document 1). This surface acoustic wave atomization device is made to perform a stable atomization with small power and efficiently by extending the liquid thinly with the liquid film formation component, while keeping a balance between a liquid supplying amount and a spraying amount.

### PRIOR ART DOCUMENT

### PATENT DOCUMENT

Patent document 1: Japanese Laid-open Patent Publication No. 2008-104974

The late published document WO 2009/145099 A1 discloses an atomization apparatus which includes a substrate, electrolysis device and a vibration device, wherein in the substrate a liquid receiving part and a reservoir is formed. The electrolysis device has two electrodes located at a liquid receiving part, which is formed as a hollow part in the substrate. Liquid is produced by condensation using a Peltier unit and after a subsequent electrolysis the produced water is moved by capillarity into the reservoir, wherein generated surface acoustic waves discharge the water.

### Disclosure of the invention

The present invention relates to an atomization device according to claim 1. Claims 2 to 9 refer to specifically advantageous realizations of the subject matter of claim 1.

However, in the surface acoustic wave atomization device shown in the patent document 1 mentioned above, the supplied liquid is atomized directly, and it is desired to provide new positive effect for the supplied liquid when atomizing. For example, for the purpose of health improvement etc., it has been made to use hydrogenated water in which hydrogen gas is dissolved or to use oxygenated water in which oxygen gas is dissolved where the gases are produced by electrolyzing the water. Since hydrogenated water has reduction ability, an anti-aging effect and a long-term storage effect for food are expected. Moreover, oxygenated water relieves insufficient oxygen of a cell by supplying oxygen, and recovery-from-fatigue effect etc. is expected. It is desired to provide such positive effect during atomization of the supplied liquid in a surface acoustic wave atomization device. Moreover, in order to build into various apparatus the device, which can provide such effect for the liquid and carry out atomization, and in order to use it with sufficient user-friendliness, it is desirable to make the whole device compact.

The present invention is to solve the above problem, and an object of the present invention is to provide a surface acoustic wave atomization device, which can generate, with a simple constitution, a mist provided with new positive effect by making gas or ions to dissolve in a supplied liquid while atomizing, and moreover, which can be constituted compactly.

A surface acoustic wave atomization device for atomizing a liquid by using energy of a surface acoustic wave, according to an aspect of the present invention, comprises: a substrate which is made of a piezoelectric material and comprises an electrode, to which a high-frequency voltage is impressed, provided on a surface of the substrate for generating the surface acoustic wave; a liquid supply unit for supplying a liquid on the substrate; a pair of electrolysis electrodes for being impressed with a voltage to electrolyze the liquid supplied from the liquid supply unit. The electrolysis electrodes are arranged facing the surface of the substrate so that the electrolysis electrodes hold the liquid between the electrolysis electrodes and between the electrolysis electrodes and the surface of the substrate by using a surface tension of the liquid. The surface acoustic wave atomization device atomizes the liquid by using the energy of the surface acoustic wave while electrolyzing the liquid.

According to such constitution, the liquid can be stably held between the electrolysis electrodes, and a mist provided with new positive effect can be generated by making gas or ions dissolve in the liquid in in-situ of atomization of the held liquid, and moreover, the device can be constituted compactly.

### BRIEF DESCRIPTION OF THE DRAWINGS

FIG. 1A is a schematic perspective view of a surface acoustic wave atomization device according to a first embodiment of the present invention, and FIG. 1B is a fragmentary sectional view of the device along the direction of the surface acoustic wave propagation.
FIG. 2A is a schematic perspective view of a surface acoustic wave atomization device according to a second embodiment, and FIG. 2B is a fragmentary sectional view of the device along the direction of the surface acoustic wave propagation.
FIG. 3 is an exploded perspective view of a working example of the device.
FIG. 4 is an assembly perspective view with a sight through part of the device shown in FIG. 3.
FIG. 5A is a schematic perspective view of a surface acoustic wave atomization device according to a third embodiment, and FIG. 5B is a fragmentary sectional view of the device along the direction of the surface acoustic wave propagation.
FIG. 6 is a perspective view of a principal part of a working example of the device.
FIG. 7 is a sectional view around a liquid holding member shown in FIG. 6.

### MODE FOR CARRYING OUT THE INVENTION

### (First embodiment)

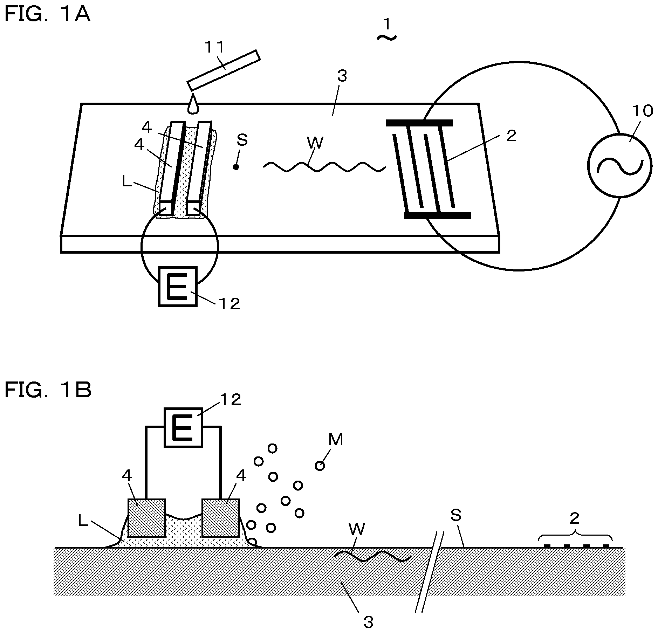

Hereafter, a surface acoustic wave atomization device according to embodiments of the present invention is described with reference to the drawings. FIG. 1A and FIG. 1B show a surface acoustic wave atomization device 1 according to the first embodiment. The surface acoustic wave atomization device 1 comprises a substrate 3 made of piezoelectric material in which a pattern electrode 2 for generating a surface acoustic wave W is formed. The substrate 3 is excited by impressing high-frequency voltage to the pattern electrode 2 from a high-frequency power source 10, and a surface acoustic wave W is generated and propagates on a surface S of the substrate 3. The surface acoustic wave atomization device 1 makes a liquid L fly as micro-particles M to atomize the liquid L by energy of the surface acoustic wave W; the liquid L is supplied from a liquid supply unit 11 to an area in the surface S where the surface acoustic wave W propagates. The surface acoustic wave atomization device 1 comprises a pair of electrolysis electrodes 4 for electrolyzing the liquid L, which is arranged facing the surface of the substrate 3. The surface acoustic wave atomization device 1 holds the liquid L between the electrolysis electrodes 4 and between electrodes 4 and the surface S of the substrate 3 by using its surface tension. The electrodes 4 just are set and held above the substrate 3 with some gap to the substrate 3. Their concrete composition is described later. The liquid L, in contact with the electrodes 4, is electrolyzed and changes into the state including gas and ions of hydrogen or oxygen by connecting DC power supply 12 to the electrodes 4, and in that state, is atomized by the surface acoustic wave W, and a mist of micro-particles M in which gas and ions are dissolved is generated.

As the piezoelectric material for constituting the substrate 3, a piezoelectric crystal itself like LiNbO₃ (lithium niobate) may be used, for example. Moreover, a non-piezoelectric substrate having on its surface a piezoelectric thin film, such as PZT thin film (lead, zirconium, titanium-alloy thin film), for example, may be sufficient as the piezoelectric material. A surface portion of the piezoelectric thin film on the substrate surface is excited and a surface acoustic wave is generated. Therefore, all that is required for the piezoelectric material for constituting the substrate 3 is to be a substrate having on its surface a piezoelectric part where the surface acoustic wave can be generated. For both the above cases, the substrate 3 works as an oscillator. The pattern electrode 2 is a electrode made up with two comb shape electrodes engaged each other and formed on the surface S of the substrate 3 (an intersection finger electrode, IDT: interdigital transducer). Each of mutually adjacent comb teeth of the pattern electrode 2 belongs to different electrode, and is arranged at a pitch of half length of a wavelength of the surface acoustic wave W. By impressing high frequency (for example, MHz band) voltage from the high frequency source 10 dedicated to high frequency voltage impression between two comb shape electrodes of the pattern electrode 2, an electrical energy is converted into a wave mechanical energy by the comb shape electrodes, and the surface acoustic wave W is generated on the surface S. Amplitude of the generated surface acoustic wave W is determined by the strength of the voltage impressed to the pattern electrode 2, and a length of wave packet of the surface acoustic wave W corresponds to a length of time the voltage is impressed. The surface acoustic wave W becomes a wave having a width corresponding to an overlap width of the teeth of the pattern electrode 2, and propagates in forward and backward direction normal to the teeth of combs. Since the pattern electrode 2 generates surface acoustic waves propagating in both forward and backward directions, a comb shape electrode for reflection may be arranged on the surface S in an opposite side to a side where the liquid L is supplied.

The liquid supply unit 11 is arranged so that the liquid L can be supplied to the area where the surface acoustic wave W propagates and where is distant from the pattern electrode 2, and preferably so that the liquid L can be supplied near the electrolysis electrodes 4. In other words, the pattern electrode 2 for surface acoustic wave generation is arranged away from an area where the liquid L, being held with the electrolysis electrodes 4, stays. Thereby, the area for holding the liquid L on the surface S of the substrate 3 can be set at a position distant from the pattern electrode 2, and therefore damage to the pattern electrode 2 can be prevented and its life can be prolonged. The liquid supply unit 11 comprises a super-thin pipe or a porous body having a capillary structure, for example, as a liquid supplying port from which the liquid L is drawn. The liquid L is water, for example, tap water, and supplied on the surface S by dropping or oozing it from the liquid supplying port of the liquid supply unit 11. In addition, the liquid L is not limited to simple water, and water in which gas such as ozone or fusible material is dissolved beforehand, and also a mixture or a solution of arbitrary liquids other than water may be used. The liquid L may be an electrolytic solution which is electrolyzed and made by adding electrolytic substance to pure water.

The electrodes 4 are two metal square bars held in parallel mutually by using a not-shown component, and arranged above the substrate 3, facing the surface S of the substrate 3 so that each forms a fixed gap to the surface S. The gap between the electrodes 4 and the gap between the electrodes 4 and the surface S may be determined on a balance of an amount of the liquid L which is supplied and held between the electrodes 4 and an amount of a liquid which oozes from the held liquid L to the pattern electrode 2 side and is consumed by atomization. The electrodes 4 are not necessary to be separated from the surface S on the whole surface, and they may touch on the surface S partly. Each of the two metal square bars for constituting the electrodes 4 is not necessary to be linear, and can be made into an arbitrary shape, such as a curved shape, a crooked zigzag shape like a V or W character, and a shape of combination of such shapes, and the pair of the electrodes 4 does not need to be arranged in parallel at equal intervals mutually. Moreover, in case that the electrodes 4 curve, the direction of the curve may be either convex or concave toward the pattern electrode 2. Preferably, as shown in FIG. 1A, the pair of the electrodes 4 is arranged so that only one of the electrodes 4 faces the pattern electrode 2, that is, the surface acoustic wave W propagates into the liquid held around one of the electrodes 4. According to such arrangement of the pair of the electrodes 4, since atomization occurs actively generally in the place where the surface acoustic wave W reaches the liquid L first after its propagation, micro-particles M can be generated, which contain ions or gas generated with one of the electrodes 4 selectively according to use of the mist as shown below.

Not only which one of the pair of the electrodes 4 is set as an anode or cathode but also material of the electrodes 4 are suitably chosen according to use of the surface acoustic wave atomization device 1. For example, the surface acoustic wave atomization device 1 may be used for generating a mist from hydrogenated water dissolving hydrogen gas generated by electrolyzing water. In this case, one of the electrolysis electrodes 4 arranged in the side of the pattern electrode 2 is set as an anode, and hydrogen gas is generated in a portion of the liquid L where the one of the electrodes 4 touches. Moreover, when the use is the use for generating a mist from a oxygenated water dissolving oxygen gas generated by electrolyzing water, one of the electrolysis electrodes 4 arranged in the side of the pattern electrode 2 is set as a cathode and oxygen gas is produced in a portion of the liquid L to which the one of the electrodes 4 touches. In such electrolysis of the liquid L, in order to suppress consumption of the electrodes 4 or to maintain purity of the liquid, metal which is hard to rust and hard to elute because of low ionization tendency, for example, platinum etc., can be used conveniently as a material of the electrodes 4. Moreover, in order to supply metal ions into the liquid L actively, the electrodes 4 of metal which is of high ionization tendency and easy to elute, for example, copper, silver, etc. may be arranged in the side of the pattern electrode 2 and used as an anode. In addition, the arrangement configuration of the electrodes 4 is not restricted to the above arrangement and an arrangement that the both electrodes 4 face the pattern electrode 2 may be good. In the case that the both electrodes 4 face the pattern electrode 2 and the surface acoustic wave W propagates into the liquid held around the both electrodes 4, depending on the material of the both electrodes 4 or the state of voltage impressed, a mist containing ions and a mist containing hydrogenated water and oxygenated water are generated in a mixed state.

According to this embodiment, since the liquid L is held by simple constitution between the pair of electrolysis electrodes 4 and between the electrodes 4 and the surface S of the substrate 3, the liquid L can be held stably. Moreover, while atomizing, by impressing voltage between the pair of electrodes 4, hydrogen gas or oxygen gas can be made to dissolve into the liquid L in in-situ of atomization so that the mist of hydrogenated water and the mist of oxygenated water can be generated. That is, according to this embodiment, the mist of hydrogenated water or the mist of oxygenated water, that is, a mist provided with new effect by alkaline water, acid water, or hydrogen contained water, etc., can be generated self-containedly, and moreover, the device can be constituted compactly. Moreover, by arranging the electrodes 4, consisting of metal which ion tends to elute, to the pattern electrode 2 side as an anode, substances of non-liquid can be made to dissolve into the liquid L and atomized, and a mist provided with effect other than the effect by alkaline water, acid water, hydrogen contained water, etc. can be generated. Disinfection effect can be acquired in a spray of the mist by generating the mist containing metal ions (hydroxide).

### (Second embodiment)

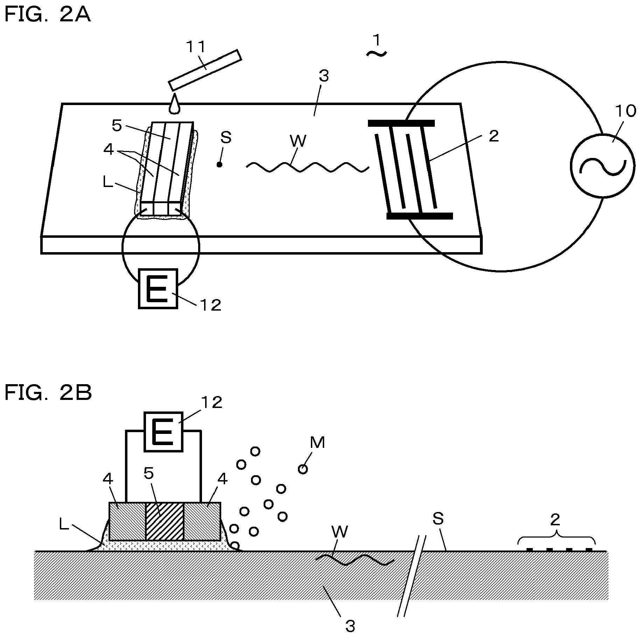

FIG. 2A and FIG. 2B show a surface acoustic wave atomization device 1 according to the second embodiment. The surface acoustic wave atomization device 1 of this embodiment is further equipped with a liquid holding member 5 arranged facing the surface S of the substrate 3 to make a gap against the surface in the surface acoustic wave atomization device 1 of the above-mentioned first embodiment. And in this embodiment, the electrolysis electrodes 4 is arranged before and behind the liquid holding member 5 in the propagation direction of the surface acoustic wave W, and others are the same as those of the first embodiment. The liquid holding member 5 has a bottom of which surface is flat and faces the surface S, and both side-walls which rise from the bottom have also flat surfaces with right angles to the bottom. The electrodes 4 are two metal square bars, and are respectively adhesion fixed to both side-walls of the liquid holding member 5. The bottom of the liquid holding member 5 and the bottom of the electrodes 4 are set flush so that a fixed gap is formed between these bottoms and the surface S of the substrate 3. The liquid L supplied by the liquid supply unit 11 is held so that it may be a constant quantity at this fixed gap. Supplying of the liquid L to the gap can be performed, for example, via through-hole (not shown) made in the liquid holding member 5 and directed to the surface S, or by using capillary phenomenon from an end side of the liquid holding member 5. In addition, the constitution of the liquid holding member 5 and the electrodes 4 is not restricted to the constitution shown in FIG. 2A and FIG. 2B, but can be made into the shape curved and crooked as well as the explanation in the first embodiment mentioned above. Moreover, without carrying out adhesion fixing of the electrodes 4 of metal square bars to the liquid holding member 5, the electrodes 4 may be metal plates fixed to the liquid holding member 5 by insertion or screw clamps, and also the electrodes 4 may be made with metal films on the surface of the liquid holding member 5 which are formed with evaporation coating, sputtering, plating, etc.. The liquid holding member 5 itself is constituted by using insulating materials, such as resin or ceramics. Moreover, the electrolysis electrodes 4 may be made, instead of the metal film forming on a surface of insulating material and patterning as mentioned above, in a integrated form with the liquid holding member 5 by co-firing of ceramic material and conductive paste or by molding of resin with insert or outsert of electrode material. Integration of the liquid holding member 5 and the electrolysis electrodes 4 into one part enables to reduce parts count, to make the part replacement easy, and to provide a compact part. According to this embodiment, by using the liquid holding member 5 provided in addition to the electrolysis electrodes, it can easily perform adjusting or restricting the quantity of the liquid more properly, and it can electrolyze or ionize a proper quantity of liquids with low electric power, and efficient atomization can be performed.

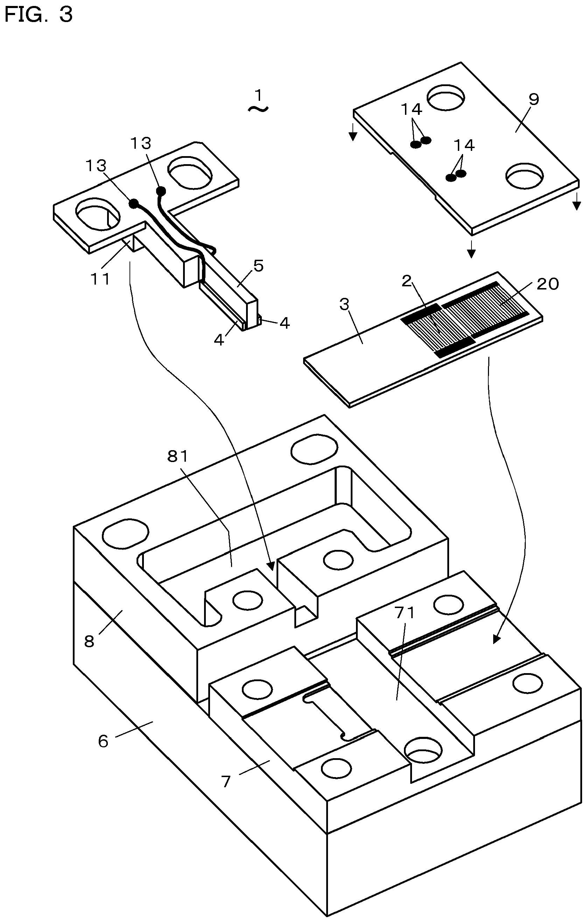

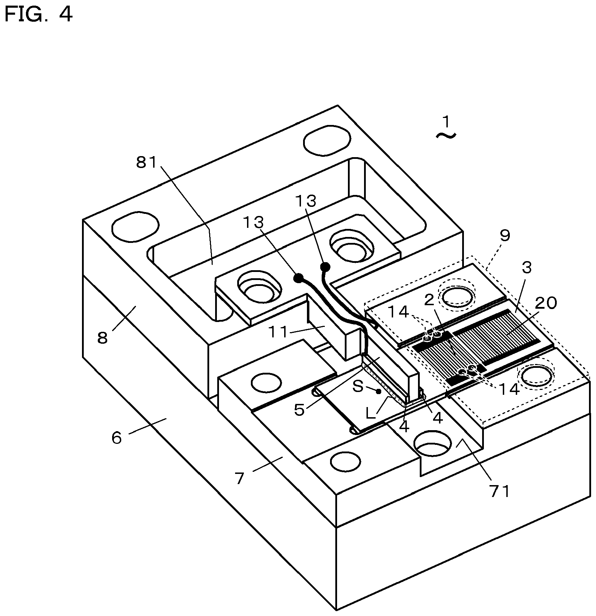

FIG. 3 and FIG. 4 show a working example of the second embodiment. This surface acoustic wave atomization device 1 comprises a substrate 3, a liquid holding member 5 having an electrodes 4, a fixing block 6 of a rectangular parallelepiped used as a base of the whole device, a buttress plate 7 arranged near by one end side on the fixing block 6, a liquid container 8, and a contact jig 9 equipped on the substrate 3. The buttress plate 7 is a basic component which supports the substrate 3 from a lower part. The liquid container 8 having a liquid reservoir part 81 is arranged near by another end side on the fixing block 6. The substrate 3 is formed in a rectangle plate. The substrate 3 comprises not only a pattern electrode 2 which generates the surface acoustic wave W, but also a comb shape reflecting electrode 20 for total reflection and effective use of a surface acoustic wave which goes in an opposite direction to a side to which a liquid L is supplied. The pattern electrode 2 is combined with the comb shape reflecting electrode 20, and constitutes a one-directional electrode which generates a surface acoustic wave W propagating to one way only. The substrate 3 is arranged on the buttress plate 7 so that it may bridge a groove 71 formed in the buttress plate 7, and fixed to the buttress plate 7 by the contact jig 9 which is fixed to the buttress plate 7 by using two screws (not shown and other screws are the same).

The contact jig 9, which has electrode pins 14 for electrically connecting and impressing voltage to the pattern electrode 2, is attached on the substrate 3 from the upper part of the pattern electrode 2 so as to be fixed to the substrate 3 and to be electrically connected to the pattern electrode 2. In addition, the contact jig 9 has a recess formed in the surface facing the substrate 3 so that generation and propagation of the surface acoustic wave W are not disturbed. The area in the substrate 3 where liquid supply and atomization are performed is set at a position of the groove 71 in the buttress plate 7, and therefore superfluous liquid is exhausted through the groove 71 and does not reach the pattern electrode 2. The liquid holding member 5 has a wings-like component having holes for fixation at an end of its rod-like form component, and is fixed to the liquid container 8 in a posture of a cantilever beam by using two screws through the wings-like component and the holes for fixation. Another end (free end) of the liquid holding member 5 is of rectangle plate, and is arranged in the standing posture facing the surface S of the substrate 3 to form a gap so that the gap constitutes a holding part holding the liquid L. In the free end side of the liquid holding member 5, electrolysis electrodes 4 is equipped, and extraction electrodes 13, which are electrically connected to the electrodes 4, is provided on a top face of the liquid holding member 5. This liquid holding member 5 is integrally constituted with a liquid supply unit 11. The liquid supply unit 11 supplies the liquid L to a holding part area in the surface S of the substrate 3 from the liquid reservoir part 81 of the liquid container 8. A lower convex part of the liquid supply unit 11 sucks up the liquid (not shown) from the liquid reservoir part 81, and the liquid migrates and is supplied at the free end side of the liquid holding member 5. For example, the capillarity phenomenon caused by unevenness or porosity given to the surface of the liquid supply unit 11 is used for sucking and migration of liquid. The surface acoustic wave atomization device 1 of this working example is realized in a compact constitution by such composition.

### (Third embodiment)

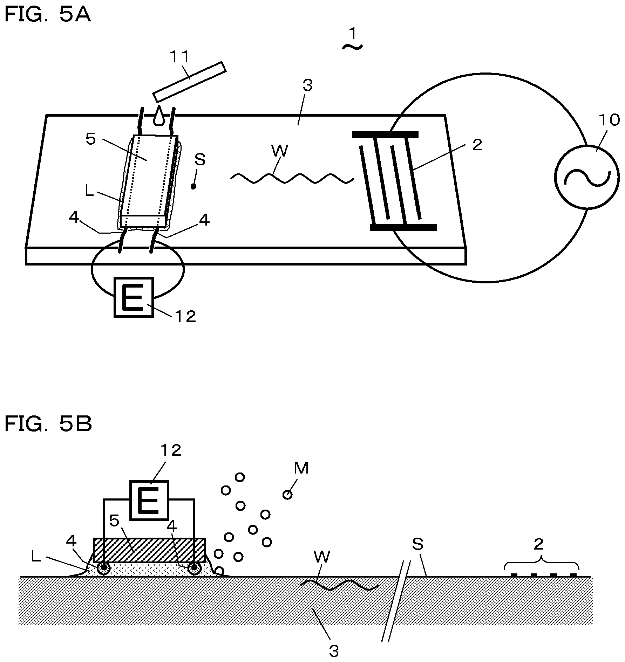

FIG. 5A and FIG. 5B show a surface acoustic wave atomization device 1 according to the third embodiment. The surface acoustic wave atomization device 1 of this embodiment is the one that the electrolysis electrodes 4 are formed with metal wires in the second embodiment mentioned above, and the others are the same as those of the second embodiment. Although the electrodes 4 made of metal wires are arranged on a bottom surface of the liquid holding member 5 in the figures, they also may be arranged on the side surfaces of the liquid holding member 5. Moreover, without using the liquid holding member 5, in the surface acoustic wave atomization device 1 of the above-described first embodiment, the liquid L may be held between the electrodes 4 made of metal wires and the surface S of the substrate 3. The metal wires for making the electrodes 4 may be chosen and used according to use from among marketed things of various sizes of circular cross section and of various material. Moreover, as the electrodes 4, the cross section is not restricted to circle, but a quadrangle and a polygon may be sufficient, and not only marketing but arbitrary metal wires may also be used. Moreover, a strand containing two or more metal wires may be used as the metal wire for making the electrodes 4. As material of the metal wire of the electrodes 4, rust resistant metal, or metal which can supply metal ions into the liquid L actively, etc. can be chosen and used according to intended use, and this point is the same as that of the second embodiment mentioned above. According to this embodiment, the electrolysis electrodes 4 can be easily manufactured at low cost by using an easily available metal wire.

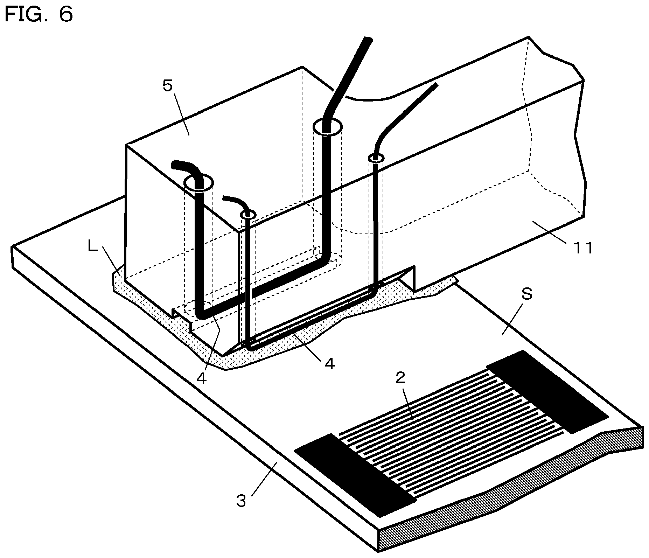

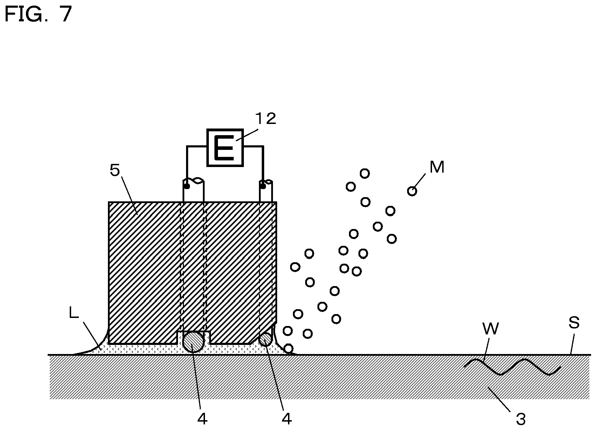

FIG. 6 and FIG. 7 show a working example of the third embodiment. This surface acoustic wave atomization device 1 holds each wire of the electrodes 4 by making in the liquid holding member 5 two through holes in the vertical direction and a groove or a chamfer at the bottom surface for connecting the through holes, and by arranging the metal wires by using them, in the surface acoustic wave atomization device 1 shown in above-mentioned FIG. 3 and FIG. 4. That is, this surface acoustic wave atomization device 1 is equipped with the metal wires as the electrodes 4 in the groove and the chamfer, in a manner that each metal wire is inserted into the one through hole from upper side, laid in the groove or the chamfer at the bottom surface, and drawn from the other through hole. The both ends of the metal wire coming out from the through holes to upper side may be fixed by a screw stop etc., and electrical connection can be carried out to the DC power supply dedicated to electrolysis. Thus, since the both ends of a metal wire are easily fixable by a screw stop etc., when the metal wire which forms the electrodes 4 is exhausted or disconnected, it is easily exchanged. Although the figures show a constitution that one of the pair electrodes 4 is arranged in the groove and another one is arranged in the chamfer, such a constitution arranging both in grooves or both in chamfers is also acceptable.

In addition, various modifications are possible for the present invention, without being restricted to the constitution mentioned above. For example, contents of each embodiment mentioned above can be combined mutually to yield other constitution. Moreover, the surface acoustic wave atomization device 1 may comprise a changeover switch for changing the positive and negative of the voltage impressed to the pair electrodes 4. The mist by oxygenated water and the mist by hydrogenated water can be easily changed by using the changeover switch. Moreover, the pair of electrolysis electrodes 4 may be composed of an inner electrode and an outer electrode in a plan view, where the outer electrode surrounds the inner electrode, and the surface acoustic wave W may be made to propagate to the outer electrode from not only one direction but two directions, or multiple directions. In this case, the pattern electrode 2 is arranged in not only one direction but two directions, or multiple directions to the outer electrode. Thereby, the amount of mist to be generated can be increased.

This application is based on the Japan patent application 2009-258091, and the contents of which are hereby incorporated by referring to the specification and figures of the above-mentioned patent application.

### EXPLANATIONS OF LETTERS OR NUMERALS

- 1: surface acoustic wave atomization device
- 2: pattern electrode
- 3: substrate
- 4: electrolysis electrodes
- 5: liquid holding member
- L: liquid
- S: surface
- W: surface acoustic wave

## Claims

1. A surface acoustic wave atomization device (1) for atomizing a liquid (L) by using energy of a surface acoustic wave (W), comprising:
a substrate (3) which is made of a piezoelectric material and comprises an electrode (2), to which a high-frequency voltage is impressed, provided on a surface (S) of the substrate (3) for generating the surface acoustic wave;
a liquid supply unit (11) for supplying a liquid on the substrate (3); wherein
the surface acoustic wave atomization device (1) atomizes the liquid (L) by using the energy of the surface acoustic wave (W) while electrolyzing the liquid (L),
**characterized in that** it comprises a pair of electrolysis electrodes (4) for being impressed with a voltage to electrolyze the liquid (L) supplied from the liquid supply unit (11), said electrolysis electrodes (4) are arranged facing the surface (S) of the substrate (3) with a fixed gap to the surface (S) so that the electrolysis electrodes (4) hold the liquid (L) between the electrolysis electrodes (4) and between the electrolysis electrodes (4) and the surface (S) of the substrate (3) by using a surface tension of the liquid (L).

2. The surface acoustic wave atomization device (1) according to claim 1, wherein the electrode (2) for generating the surface acoustic wave (W) is arranged away from an area in which the liquid (L) is held with the electrolysis electrodes (4) and stays.

3. The surface acoustic wave atomization device (1) according to claim 1, wherein each of the pair of electrolysis electrodes (4) is arranged in parallel mutually, and one electrode of them is arranged facing the electrode (2) for generating the surface acoustic wave (W).

4. The surface acoustic wave atomization device (1) according to claim 1, wherein the electrode (2) for generating the surface acoustic wave (W) is arranged away from an area in which the liquid (L) is held with the electrolysis electrodes and stays, and
each of the pair of electrolysis electrodes (4) is arranged in parallel mutually, and one electrode of them is arranged facing the electrode (2) for generating the surface acoustic wave (W).

5. The surface acoustic wave atomization device (1) according to claim 4, wherein one electrode of the electrolysis electrodes (4) is set as an anode, and generates hydrogen gas in the liquid.

6. The surface acoustic wave atomization device (1) according to claim 4, wherein one electrode of the electrolysis electrodes (4) is set as a cathode, and generates oxygen gas in the liquid.

7. The surface acoustic wave atomization device (1) according to claim 1, wherein the electrolysis electrodes (4) are made of metal wires.

8. The surface acoustic wave atomization device (1) according to claim 1, wherein the electrolysis electrodes (4) supply metal ions into the liquid (L) held with the electrolysis electrodes impressed with a voltage.

9. The surface acoustic wave atomization device (1) according to one of claims 1 to 8, wherein
the electrolysis electrodes (4) are arranged before and behind mutually in the propagation direction of the surface acoustic wave (W), and wherein
the surface acoustic wave atomization device (1) further comprises a liquid holding member (5) which faces the surface (S) of the substrate (3) and is arranged between the electrolysis-electrodes (4) so as to make a gap for holding the liquid (L).

## Patentansprüche

1. Akustische Oberflächenwellen-Zerstäubungsvorrichtung (1) zum Zerstäuben einer Flüssigkeit (L) unter Verwendung von Energie einer akustischen Oberflächenwelle (W), die umfasst:
ein Substrat (3), welches aus einem piezoelektrischen Material hergestellt ist und eine auf einer Oberfläche (S) des Substrats (3) vorgesehene Elektrode (2) umfasst, an welche eine Hochfrequenzspannung angelegt wird, um die akustische Oberflächenwelle zu erzeugen;
eine Flüssigkeitszufuhreinheit (11) zum Zuführen einer Flüssigkeit auf das Substrat (3);
wobei die akustische Oberflächenwellen-Zerstäubungsvorrichtung (1) die Flüssigkeit (L) unter Verwendung der Energie der akustischen Oberflächenwelle (W) zerstäubt, während die Flüssigkeit (L) elektrolysiert wird,
**dadurch gekennzeichnet, dass**
sie ein Paar Elektrolyseelektroden (4) umfasst, an die eine Spannung anzulegen ist, um die von der Flüssigkeitszufuhreinheit (11) zugeführte Flüssigkeit (L) zu elektrolysieren, wobei die Elektrolyseelektroden (4) der Oberfläche (S) des Substrats (3) gegenüberliegend mit einer festen Lücke zu der Oberfläche (S) angeordnet sind, so dass die Elektrolyseelektroden (4) die Flüssigkeit (L) unter Nutzung einer Oberflächenspannung der Flüssigkeit (L) zwischen den Elektrolyseelektroden (4) und zwischen den Elektrolyseelektroden (4) und der Oberfläche (S) des Substrats (3) halten.

2. Akustische Oberflächenwellen-Zerstäubungsvorrichtung (1) nach Anspruch 1, wobei die Elektrode (2) zum Erzeugen der akustischen Oberflächenwelle (W) von einem Bereich entfernt angeordnet ist, in welchem die Flüssigkeit (L) mit den Elektrolyseelektroden (4) gehalten wird und bleibt.

3. Akustische Oberflächenwellen-Zerstäubungsvorrichtung (1) nach Anspruch 1, wobei die beiden Elektrolyseelektroden (4) des Paars parallel zueinander angeordnet sind und eine Elektrode von diesen der Elektrode (2) zum Erzeugen der akustischen Oberflächenwelle (W) gegenüberliegend angeordnet ist.

4. Akustische Oberflächenwellen-Zerstäubungsvorrichtung (1) nach Anspruch 1, wobei die Elektrode (2) zum Erzeugen der akustischen Oberflächenwelle (W) von einem Bereich entfernt angeordnet ist, in welchem die Flüssigkeit (L) mit den Elektrolyseelektroden gehalten wird und bleibt, und
die beiden Elektrolyseelektroden (4) des Paars parallel zueinander angeordnet sind und eine Elektrode von diesen der Elektrode (2) zum Erzeugen der akustischen Oberflächenwelle (W) gegenüberliegend angeordnet ist.

5. Akustische Oberflächenwellen-Zerstäubungsvorrichtung (1) nach Anspruch 4, wobei eine Elektrode der Elektrolyseelektroden (4) als eine Anode eingestellt ist und Wasserstoffgas in der Flüssigkeit erzeugt.

6. Akustische Oberflächenwellen-Zerstäubungsvorrichtung (1) nach Anspruch 4, wobei eine Elektrode der Elektrolyseelektroden (4) als eine Kathode eingestellt ist und Sauerstoffgas in der Flüssigkeit erzeugt.

7. Akustische Oberflächenwellen-Zerstäubungsvorrichtung (1) nach Anspruch 1, wobei die Elektrolyseelektroden (4) aus Metalldrähten hergestellt sind.

8. Akustische Oberflächenwellen-Zerstäubungsvorrichtung (1) nach Anspruch 1, wobei die Elektrolyseelektroden (4) Metallionen in die Flüssigkeit (L) zuführen, die mit den Elektrolyseelektroden gehalten wird, an die eine Spannung angelegt ist.

9. Akustische Oberflächenwellen-Zerstäubungsvorrichtung (1) nach einem der Ansprüche 1 bis 8, wobei
die Elektrolyseelektroden (4) als vordere und hintere in der Fortpflanzungsrichtung der akustischen Oberflächenwelle (W) angeordnet sind und wobei
die akustische Oberflächenwellen-Zerstäubungsvorrichtung (1) ferner ein Flüssigkeitshalteelement (5) umfasst, welches der Oberfläche (S) des Substrats (3) gegenüberliegt und so zwischen den Elektrolyseelektroden (4) angeordnet ist, dass eine Lücke zum Halten der Flüssigkeit (L) erzeugt wird.

## Revendications

1. Dispositif d'atomisation à onde acoustique de surface (1) pour atomiser un liquide (L) en utilisant l'énergie d'une onde acoustique de surface (W), comprenant :
un substrat (3) qui est constitué d'un matériau piézoélectrique et qui comprend une électrode (2), à laquelle une tension haute fréquence est appliquée, prévue sur une surface (S) du substrat (3) pour générer l'onde acoustique de surface ;
une unité de fourniture de liquide (11) pour fournir un liquide sur le substrat (3) ;
dans lequel
le dispositif d'atomisation à onde acoustique de surface (1) atomise le liquide (L) en utilisant l'énergie de l'onde acoustique de surface (W) tout en électrolysant le liquide (L),
**caractérisé en ce que**
il comprend une paire d'électrodes d'électrolyse (4) auxquelles une tension est appliquée pour électrolyser le liquide (L) fourni par l'unité de fourniture de liquide (11), lesdites électrodes d'électrolyse (4) sont agencées faisant face à la surface (S) du substrat (3) avec un espace fixe par rapport à la surface (S) de sorte que les électrodes d'électrolyse (4) maintiennent le liquide (L) entre les électrodes d'électrolyse (4) et entre les électrodes d'électrolyse (4) et la surface (S) du substrat (3) en utilisant une tension superficielle du liquide (L).

2. Dispositif d'atomisation à onde acoustique de surface (1) selon la revendication 1, dans lequel l'électrode (2) pour générer l'onde acoustique de surface (W) est agencée à distance d'une zone dans laquelle le liquide (L) est maintenu par les électrodes d'électrolyse (4) et reste.

3. Dispositif d'atomisation à onde acoustique de surface (1) selon la revendication 1, dans lequel chacune de la paire d'électrodes d'électrolyse (4) est agencée parallèlement à l'autre, et l'une de celles-ci est agencée face à l'électrode (2) pour générer l'onde acoustique de surface (W).

4. Dispositif d'atomisation à onde acoustique de surface (1) selon la revendication 1, dans lequel l'électrode (2) pour générer l'onde acoustique de surface (W) est agencée à distance d'une zone dans laquelle le liquide (L) est maintenu par les électrodes d'électrolyse et reste, et
chacune de la paire d'électrodes d'électrolyse (4) est agencée parallèlement à l'autre, et l'une de celles-ci est agencée face à l'électrode (2) pour générer l'onde acoustique de surface (W).

5. Dispositif d'atomisation à onde acoustique de surface (1) selon la revendication 4, dans lequel une électrode des électrodes d'électrolyse (4) est établie en tant qu'anode, et génère de l'hydrogène gazeux dans le liquide.

6. Dispositif d'atomisation à onde acoustique de surface (1) selon la revendication 4, dans lequel une électrode des électrodes d'électrolyse (4) est établie en tant que cathode, et génère de l'oxygène gazeux dans le liquide.

7. Dispositif d'atomisation à onde acoustique de surface (1) selon la revendication 1, dans lequel les électrodes d'électrolyse (4) sont constituées de fils métalliques.

8. Dispositif d'atomisation à onde acoustique de surface (1) selon la revendication 1, dans lequel les électrodes d'électrolyse (4) fournissent des ions métalliques dans le liquide (L) maintenu par les électrodes d'électrolyse auxquelles une tension est appliquée.

9. Dispositif d'atomisation à onde acoustique de surface (1) selon l'une des revendications 1 à 8, dans lequel
les électrodes d'électrolyse (4) sont mutuellement agencées avant et derrière dans la direction de propagation de l'onde acoustique de surface (W), et dans lequel
le dispositif d'atomisation à onde acoustique de surface (1) comprend en outre un élément de maintien de liquide (5) qui est face à la surface (S) du substrat (3) et qui est agencé entre les électrodes d'électrolyse (4) de manière à réaliser un espace pour maintenir le liquide (L).
